# EUROPEAN PATENT APPLICATION

(11) **EP 1 860 088 A1**
(43) Date of publication of application: **28.11.2007**
(21) Application number: 06252729.6
(22) Date of filing: 25.05.2006
(51) Int. Cl.: C07C 29/151, C07C 31/02, C07C 31/04, C07C 31/08, C07C 31/10, C07C 31/12, C01B 3/36, C01B 3/38

(54) **Process for hydrogen, CO2 and alcohol production**

(71) Applicant: BP Chemicals Limited, Sunbury-on-Thames, Middlesex TW16 7BP (GB)
(72) Inventor: The designation of the inventor has not yet been filed
(74) Representative: De Kezel, Eric

(57) **Abstract**

This invention relates to the production of alcohol, CO2 and hydrogen from a hydrogen and carbon containing compound, such as a hydrocarbon. More specifically, this invention relates to the production from a hydrogen and carbon containing compound of
- mixed alcohols for hydrogen and CO2 improved separation and recovery,
- CO2 for hydrocarbon improved recovery from wells with optional CO2 sequestration in the wells,
- Alcohols and CO2 for enhanced hydrocarbon recovery from wells through the introduction of a stream containing the said alcohols and CO2 into the wells with optional sequestration of CO2, and
- hydrogen for power generation.

## Description

This invention relates to the production of alcohol, CO2 and hydrogen from a hydrogen and carbon containing compound, such as a hydrocarbon. More specifically, this invention relates to the production from a hydrogen and carbon containing compound of
- mixed alcohols for hydrogen and CO2 improved separation and recovery,
- CO2 for hydrocarbon improved recovery from wells with optional CO2 sequestration in the wells,
- Alcohols and CO2 for enhanced hydrocarbon recovery from wells through the introduction of a stream containing the said alcohols and CO2 into the wells with optional sequestration of CO2, and
- hydrogen for energy generation (e.g. power and heat generation and fuel cells) and/or chemical use.

US 4,122,110 relates to a process for manufacturing alcohols, particularly linear saturated primary alcohols, by reacting carbon monoxide with hydrogen at a pressure between 20 and 250 bars and a temperature between 150 DEG and 400 DEG C., in the presence of a catalyst, characterized in that the catalyst contains at least 4 essential elements: (a) copper (b) cobalt (c) at least one element M selected from chromium, iron, vanadium and manganese, and (d) at least one alkali metal.

US 4,831,060 relates to the production of mixed alcohols from carbon monoxide and hydrogen gases using a catalyst, with optionally a co-catalyst, wherein the catalyst metals are molybdenum, tungsten or rhenium, and the co-catalyst metals are cobalt, nickel or iron. The catalyst is promoted with a Fischer-Tropsch promoter like an alkali or alkaline earth series metal or a smaller amount of thorium and is further treated by sulfiding. The composition of the mixed alcohols fraction can be selected by selecting the extent of intimate contact among the catalytic components.

Journal of Catalysis 114, 90-99 (1988) discloses a mechanism of ethanol formation from synthesis gas over CuO/ZnO/Al2O3. The formation of ethanol from CO and H2 over a CuO/ZnO methanol catalyst is studied in a fixed-bed microreactor by measuring the isotopic distribution of the carbon in the product ethanol when 13C methanol was added to the feed.

The combustion of fossil fuels to generate electrical power and/or pressurised steam results in the formation of carbon dioxide, which is a so-called greenhouse gas. It is becoming increasingly desirable to minimise atmospheric emissions of such greenhouse gases to the atmosphere in order to reduce the potential harm that such greenhouse gases may have on the global climate.

Increasing attention is being focussed on hydrogen as a fuel, as the energy produced per unit mass is high, and the only combustion product is water. However, most hydrogen currently produced is derived from fossil fuels, for example from refining processes such as catalytic reforming, through processes such as steam reforming or autothermal reforming of hydrocarbons or oxygenated hydrocarbons, or through partial oxidation of hydrocarbons.

Processes describing the production of hydrogen from carbon-based fuels include, for example, that described in WO 02/45838, which relates to an apparatus having a plurality of modules for the production of substantially pure hydrogen from a hydrocarbon fuel, in which the apparatus comprises a module with a steam reforming and partial oxidation catalyst to produce hydrogen, carbon dioxide and carbon monoxide, and additional modules for high temperature and low temperature water gas shift reactions for converting carbon monoxide to carbon dioxide. Also provided is a module for sulphur removal, and a module for low temperature oxidation to remove residual traces of carbon monoxide. The resulting hydrogen-rich gas can be fed to a fuel cell for power and/or electricity generation, either with or without prior purification to remove other components such as carbon dioxide, water and residual hydrocarbon.

The separation of hydrogen from other gases can be achieved using a selectively permeable membrane. For example, US 4,810,485 describes a reactor for a hydrogen-forming reaction, for example a steam reforming or water-gas-shift reaction, which comprises a hydrogen-ion porous foil, such as a palladium foil. This is capable of selectively removing hydrogen produced in the hydrogen-forming reaction, which shifts the equilibrium and enables higher conversions of hydrogen-containing reactant and higher hydrogen yields to be achieved.

WO02/70402 describes a further reactor for the reforming of a vapourisable hydrocarbon to produce hydrogen and carbon dioxide using a reactor with a hydrogen-permeable membrane, and which is heated by flameless distributed combustion in a region of the reactor separate to that in which the steam-reforming and hydrogen separation processes occur. US 5,741,474 also describes the production of high-purity hydrogen by feeding hydrocarbon, water and oxygen to a reactor comprising a catalyst for steam reforming and partial oxidation, in which hydrogen is separated within the reactor by use of selective hydrogen-permeable membrane tubes, wherein the permeated hydrogen is combined with a low pressure inert gas to remove or flush it from the tubes.

Itoh et al in Catalysis Today, 2003, vol 82, pp119-125 describe that, in a process for dehydrogenation of cyclohexane using a palladium-membrane reactor for selectively removing hydrogen, the rate of dehydrogenation and the rate of hydrogen recovery is enhanced when the pressure difference across the membrane is increased, and states that it is advantageous to maintain the pressure on the permeate-side of the membrane as low as possible in order to improve the rate of hydrogen production. This is corroborated by the teaching of US 4,810,485, which describes how increasing hydrogen permeation rate is achievable with increased pressure-drop across the hydrogen separation membrane.

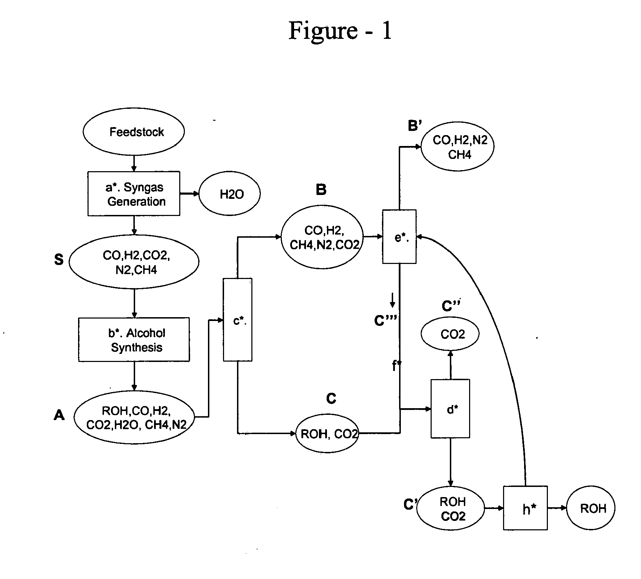

Figures 1 represents one embodiment of a process scheme according to the present invention. This said embodiment comprises optional and/or preferred process steps according to the present invention. The letter references in Figure 1 correspond to those used in the present description and appending claims.

Thus, according to the present invention, there is provided a process for the production of alcohol, CO2 and hydrogen from a hydrogen and carbon containing compound, which process comprises the steps of;
a) converting in a syngas reactor a hydrogen and carbon containing compound into a stream S consisting of a mixture of carbon oxide(s) and hydrogen, CH4 and optionally N2,
b) converting in a alcohol synthesis reactor a carbon oxide(s) and hydrogen feed from stream S into a alcohol stream A comprising alcohols, H2 and CO, CO2, H2O, CH4 and optionally N2,
c) separating the alcohol stream A into a stream B comprising essentially H2, CO, most of the CO2, CH4 and optionally N2, and a stream C comprising the alcohols and the remaining CO2,
d) separating stream C into a purified alcohol feedstock C' and a CO2 feedstock C",
e) backwashing stream B with alcohol taken from feedstock C' and recovering a stream C''' comprising essentially alcohol and CO2 and a hydrogen stream B' comprising essentially H2, CO, CH4 and optionally N2, and
f) introducing the recovered stream C"' into step d).

It is thus now possible according to the integrated process of the present invention to co-produce in an efficient process an alcohol (C'), CO2 (C") and H2 (B') feedstock from a hydrogen and carbon containing compound. Part of the alcohol is advantageously used in the integrated process in order to isolate the CO2 (C"') from the H2 rich feedstock (B).

According to an embodiment of the present invention, at least part, preferably all of the remaining alcohol can be sold or further dehydrated into olefins.

As indicated, step f consists in introducing the recovered stream C'" into step d). This can be done by introducing C"' separately from the stream C into the separator step d) or, preferably by adding stream C"' to stream C and introducing the combined stream into the separation step d).

The isolated CO2 can advantageously be used for hydrocarbon (oil and/or gas) improved recovery from wells. This recovery of hydrocarbon, e.g. oil, can even be further enhanced by introducing a stream containing the said CO2 together with some of the produced alcohols into the wells.

Optionally, the recovered CO2 can be sequestrated for long term storage into wells.

Finally, the H2 feedstock can advantageously be used for energy generation (e.g. power and heat generation and fuel cells) and/or chemical use.

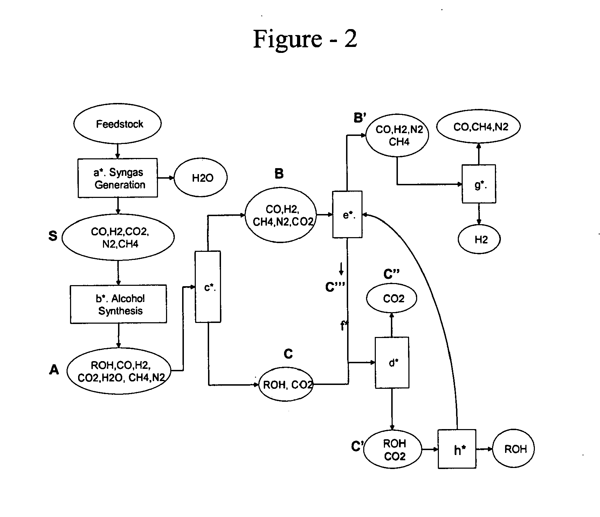

Figures 2 represents another embodiment of a process scheme according to the present invention. This said embodiment comprises optional and/or preferred process steps according to the present invention. The letter references in Figure 2 correspond to those used in the present description and appending claims. In the said Figure 2 it can be seen that the hydrogen stream B' which also comprises CO, CH4 and optionally N2, is subjected to an additional separation step g) wherein a purified H2 feedstock and a CO, CH4 and optionally N2 stream are separately recovered.

Thus, according to a preferred embodiment of the present invention, there is provided a process for the production of alcohol, CO2 and hydrogen from a hydrogen and carbon containing compound, which process comprises the steps of;
a) converting in a syngas reactor a hydrogen and carbon containing compound into a stream S consisting of a mixture of carbon oxide(s) and hydrogen, CH4 and optionally N2,
b) converting in a reactor a carbon oxide(s) and hydrogen feed from stream S into a alcohol stream A comprising alcohols, H2 and CO, CO2, H2O, CH4 and optionally N2,
c) separating the alcohol stream A into a stream B comprising essentially H2, CO, most of the CO2, CH4 and optionally N2, and a stream C comprising the alcohols and the remaining CO2,
d) separating stream C into a purified alcohol feedstock C' and a CO2 feedstock C",
e) backwashing stream B with alcohol taken from feedstock C' and recovering a stream C'" comprising essentially alcohol and CO2 and a hydrogen stream B' comprising essentially H2, CO, CH4 and optionally N2,
f) introducing the recovered stream C"' into step d), and
g) separating hydrogen stream B' into a purified hydrogen feedstock and a separated CO, CH4 and optionally N2 stream.

Any hydrogen and carbon containing compound that can be converted into a feedstock comprising carbon oxide(s) and hydrogen, most preferably into a synthesis gas (or "syngas"), is useful as primary feedstock in the processes of the invention.

The hydrogen and carbon containing compound (hydrocarbon feedstock) used for syngas generation is preferably a carbonaceous material, for example biomass, plastic, naphtha, refinery bottoms, smelter off gas, municipal waste, coal, coke and/or natural gas, coal and natural gas being the preferred ones, most preferably natural gas.

Natural gas, the most preferred hydrocarbon feedstock used according to the process of the present invention, usually contains varying amounts of N2 and other inert gases (e.g. He, Ar, ...) that make some well fields economically unattractive. For example it can necessitate a higher purge from a methanol process. Similarly, natural gas can also contain CO2 which again make some well fields economically unattractive. An advantage according to the process of the present invention is that the present process can cope with the said N2 and/or CO2 presences by advantageously recovering the said N2 and/or the said CO2 and, for example, re-injecting the said N2 and/or the said CO2 into the well field in order to improve the natural gas recovery; this makes more fields economically attractive and also increase the lifetime thereof. For example, from the H2 recovery stage g, the N2 stream can also be used to enhance hydrocarbon recovery from well.

While natural gas is the most preferred feedstock used according to the present invention, coke, coal biomass and refinery bottoms can also advantageously be used according to an embodiment of the present invention. Indeed, while the use of the said coke, coal biomass and refinery bottoms feedstocks generates sub stoichiometry syngas, the corresponding CO2 sequestration allows economic use thereof with lowered environmental impact.

Processes for producing mixtures of carbon oxide(s) and hydrogen (synthesis gas) are well known. Each has its advantages and disadvantages and the choice of using a particular reforming process is dictated by economic and available feed stream considerations, as well as by the desired mole ratio of H2:CO in the feedstock resulting from the reforming reaction. The synthesis gas may be prepared using any of the processes known in the art including partial oxidation of hydrocarbons (POX), steam reforming (SR), advanced gas heated reforming (AGHR), microchannel reforming (as described in, for example, US 6,284,217), plasma reforming, autothermal reforming (ATR) and any combination thereof. A discussion of these synthesis gas production technologies is provided in "Hydrocarbon Processing" V78, N.4, 87-90, 92-93 (April 1999) and "Petrole et Techniques", N. 415, 86-93 (July-August 1998). It is also envisaged that the synthesis gas may be obtained by catalytic partial oxidation of hydrocarbons in a microstructured reactor as exemplified in "IMRET 3: Proceedings of the Third International Conference on Microreaction Technology", Editor W Ehrfeld, Springer Verlag, 1999, pages 187-196. Alternatively, the synthesis gas may be obtained by short contact time catalytic partial oxidation of hydrocarbonaceous feedstocks as described in EP 0303438. Preferably, the synthesis gas is obtained via a "Compact Reformer" process as described in "Hydrocarbon Engineering", 2000, 5, (5), 67-69; "Hydrocarbon Processing", 79/9, 34 (September 2000); "Today's Refinery", 15/8, 9 (August 2000); WO 99/02254; and WO 200023689.

According to an embodiment of the present invention, the syngas used in the present invention is obtained via a methane steam reforming (MSR) or via the combination of methane steam reforming with a partial oxidation of hydrocarbons.

According to another embodiment of the present invention, the syngas used in the present invention is obtained via a autothermal reforming (ATR) process; indeed, the use of a large scale ATR technology makes the overall economics of the process according to the present invention even more attractive.

Optionally the overall carbon footprint that is the emissions to the environment can be further reduced by sequestrating the CO2 generated by the use of fuel for the SMR and/or ATR/SMR syngas generation units.

Typically, for commercial syngas production the pressure at which the synthesis gas is produced ranges from approximately 2 to 100 bar, preferably 20 to 75 bar and the temperature at which the synthesis gas exits the reformer ranges from approximately 700 DEG C. to 1100°C. The synthesis gas contains a molar ratio of hydrogen to carbon oxide - which is dependent on the hydrocarbon feedstock - ranging from 0.8 to 3.

According to an embodiment of the present invention, the (H2-CO2):(CO+CO2) molar ratio of stream S obtained from the syngas reactor when natural gas is used is of at least 0.8, preferably between 1.0 and 3.0 and most preferably between 2.4 and 2.8.

According to an embodiment of the present invention, the (H2-CO2):(CO+CO2) molar ratio of the carbon oxide(s) and hydrogen feed from stream S introduced into the alcohol synthesis reactor is comprised between 0.5 and 2.0, preferably comprised between 0.8 and 1.3. The potential excess of hydrogen can usefully be used for energy generation (e.g. power and heat generation and fuel cells) and/or chemical use. For example, this excess hydrogen can easily be separated from stream S by using a membrane or a cryogenic separation if high purity hydrogen is required.

According to an embodiment of the present invention, the alcohols produced in step b) are mainly methanol, propanol, ethanol and butanols (predominately n- butanol and isobutanol); said methanol, propanols ( predominately n-propanol with low amounts of isopropanol) ethanol and butanol preferably represent together at least 50% by weight of the liquid products obtained from the alcohol synthesis reactor, more preferably at least 75% by weight, most preferably at least 90% by weight.

According to another embodiment of the present invention, water and carbon dioxide are also produced in the reactor; and the water and alcohols preferably represent together at least 80% by weight of the liquid products obtained from the reactor, preferably at least 90wt%, more preferably at least 95wt%, most preferably at least 99wt%.

The particulate catalyst according to the present invention for the conversion of carbon oxide(s) and hydrogen containing feedstocks, e.g. synthesis gas or syngas, to alcohols is preferably a particulate modified molybdenum sulphide based catalyst and/or a modified methanol based catalyst and/or a modified Fischer-Tropsch catalyst and/or a rhodium-based heterogeneous catalyst.

Preferably, the catalyst used in the alcohol synthesis reactor contains at least molybdenum and/or copper; it is preferably promoted by the addition of an alkali metal salt.

Molybdenum sulphide based catalysts are preferred; a salt of potassium, especially potassium carbonate, is the preferred promoter.

Most preferably, the catalyst used is a molybdenum sulphide based catalysts containing cobalt, the molybdenum to cobalt molar ratio being preferably comprised between 1.5 and 2.5, more preferably 2; the said molybdenum sulphide based catalysts containing cobalt is most preferably promoted with potassium carbonate.

According to an embodiment of the present invention, for the Molybdenum based catalyst, the temperature in the alcohol synthesis reaction zone is selected from the range of starting from about 200°C to about 400°C. The gas hourly space velocity (GHSV) of the feedstock (litres of feedstock/hr/litre of catalyst) passing through the reaction zone can vary significantly, depending upon a variety of factors such as, for example, reaction conditions, composition of the feedstock and quantity and type of catalyst being used. Preferably, the GHSV can be maintained at any rate in the range of from about 1 to about 30,000 hr-1 or more, preferably will be maintained at a rate of at least about 500 hr-1, and more preferably will be maintained at a rate of at least 1,000 hr-1. The pressure in the reaction zone may be selected from the range of from about 50 to 200 bar, preferably a pressure in the range of from about 80 to 150 bar. The hydrogen and carbon monoxide partial pressures should be sufficient to enable the production of the alcohols. Hydrogen and carbon monoxide may be fed separately to the conversion reactor or, preferably in combination, e.g., as synthesis gas. For purposes of this invention, GHSV is gas hourly space velocity which is the rate of gas flow over the catalyst. It is determined by dividing the volume of gas (at 25°C. and 1 atmosphere) which passes over the catalyst in one hour by the volume of the catalyst. LHSV is liquid hourly space velocity which is the rate that the liquid organic substrate is fed to the conversion reactor. It is determined by dividing the liquid volume pumped in one hour by the volume of catalyst.

The conversion to alcohols reaction can be carried out by passing the mixture of hydrogen and carbon monoxide over the conversion catalyst as a vapour phase reaction or as a liquid phase reaction, e.g., slurry reaction or trickle bed fluidized bed reactor.

The reaction may be carried out in any appropriate reactor, e.g. a tubular reactor using a fixed bed of the catalyst. The reactants may be fed to the catalyst by feeding down or up, or a combination of both, to a fixed bed located in a tubular reactor. It may be desirable to use a reactor design that operates by plug flow and causes minimal turbulence in the reactor zone. The reaction may be effected in a dynamic bed of the catalyst. In such a reaction, the bed of catalyst is moving such as in the case of a fluid bed of the catalyst. The alcohols conversion reactor may preferably be chosen amongst tubular, multitubular, slurry, moving bed, fluidised bed, radial bed, multibed or reactive distillation reactor. It is preferably a multibed or multitubular vapour phase reactor.

The exit gas stream from the step b) alcohol synthesis reactor can be modified by adding water and performing a water gas shift reaction to generate CO2 and H2, both of which being the added value products obtained according to the present process. This additional WGS reaction can be conveniently conducted in the alcohol synthesis reactor by adding water to a WGS active catalyst; alternatively, this can be conducted in a separate reaction stage on process streams A and/or B and/or B', preferably on streams A and/or B.

According to an embodiment of the present invention, the hot stream A from the alcohol synthesis reactor is cooled to facilitate the separation (step c) of the alcohols (stream C) from the gas stream B by using a knockout drum or a dephlegmator. This separation is preferably conducted at the alcohol synthesis reaction pressure.

The said stream B is then backwashed with alcohol taken from feedstock C', allowing to recover a stream C'" comprising essentially alcohol and CO2 and a hydrogen rich stream B' comprising essentially H2, CO, CH4 and optionally N2. The efficiency of the alcohol backwash can be improved by pre-chilling the alcohol taken from feedstock C'. An additional optional improvement to reduce the alcohol potentially entrained with the hydrogen rich stream B' is to use a fraction of C2+ alcohols (from ethanol to higher alcohols) instead of methanol containing alcohols; the preferred alcohol range used for performing the backwash is ethanol and/or propanol. Such a stream can be conveniently obtained by conventional distillation, e.g. as shown as stage h) in figures 1 and 2.

The backwashing requires good gas/liquid contact. Common methods used include vessel internals which increase internal surface area of contact between liquid and gas, such as packings, structured packings, baffles, sieve plates.

According to an alternative embodiment of the present invention, steps c) and e) can advantageously be combined into a single vessel.

As shown in Figure 2, stream B' can be additionally treated to generate a purified hydrogen feedstock and a separated CO, CH4 and optionally N2 stream. Said treatment can be performed by using a membrane separation technique. For very high purity hydrogen generation (e.g. chemical use), a cryogenic stage may also be employed according to the present invention.

The streams B and/or B' and/or B" can be used in their entirety as a fuel, e.g. for power generation and/or syngas. However, treatment e) and g) are advantageously used to reduce the undesirable CO2 emissions.

The function of stage d) is to separate stream C and stream C"' into a alcohol feedstock C' and a CO2 feedstock C". The CO2 feedstock C" is preferably produced at superatmospheric pressure in order to meet the feed requirement of chemical and sequestration processes. The degassing of the mixture is conveniently conducted by heating the mixture to reduce the solubility of the CO2 in the alcohols.

This is preferably done by degassing the mixture at intermediate pressures between the alcohol synthesis pressure and the atmospheric pressure; this can be done in several consecutive stages; the CO2 gas obtained from the lower pressure stage may require compression.

In an embodiment of the present invention, the carbon dioxide produced by the process (for example in the alcohol synthesis and/or in the fuel side of the syngas generation) is sequestered and stored so that it is not released into the atmosphere. Preferably this is achieved by feeding the carbon dioxide into an oil and/or gas well, which ensures that the carbon dioxide is unlikely to be released to the atmosphere, while simultaneously enabling improved extraction of oil and/or gas therefrom.

The carbon dioxide is preferably dried before sequestration to prevent potential corrosion problems. This is typically achieved by cooling the wet carbon dioxide stream to ambient temperature, typically below 50°C, preferably below 40°C, and feeding it to a water separator, in which the water condenses and a stream of dry carbon dioxide is produced. The condensed water can optionally be re-used in the process, for example as a feed to a pressurised steam supply or as a feed to one or more of the reactors.

## Claims

1. Process for the production of alcohol, CO2 and hydrogen from a hydrogen and carbon containing compound, which process comprises the steps of;
a) converting in a syngas reactor a hydrogen and carbon containing compound into a stream S consisting of a mixture of carbon oxide(s) and hydrogen, CH4 and optionally N2,
b) converting in a alcohol synthesis reactor a carbon oxide(s) and hydrogen feed from stream S into a alcohol stream A comprising alcohols, H2 and CO, CO2, H2O, CH4 and optionally N2,
c) separating the alcohol stream A into a stream B comprising essentially H2, CO, most of the CO2, CH4 and optionally N2, and a stream C comprising the alcohols and the remaining CO2,
d) separating stream C into a purified alcohol feedstock C' and a CO2 feedstock C",
e) backwashing stream B with alcohol taken from feedstock C' and recovering a stream C'" comprising essentially alcohol and CO2 and a hydrogen stream B' comprising essentially H2, CO, CH4 and optionally N2, and
f) introducing the recovered stream C"' into step d).

2. Process for the production of alcohol, CO2 and hydrogen from a hydrogen and carbon containing compound, which process comprises the steps of;
a) converting in a syngas reactor a hydrogen and carbon containing compound into a stream S consisting of a mixture of carbon oxide(s) and hydrogen, CH4 and optionally N2,
b) converting in a reactor a carbon oxide(s) and hydrogen feed from stream S into a alcohol stream A comprising alcohols, H2 and CO, CO2, H2O, CH4 and optionally N2,
c) separating the alcohol stream A into a stream B comprising essentially H2, CO, most of the CO2, CH4 and optionally N2, and a stream C comprising the alcohols and the remaining CO2,
d) separating stream C into a purified alcohol feedstock C' and a CO2 feedstock C",
e) backwashing stream B with alcohol taken from feedstock C' and recovering a stream C'" comprising essentially alcohol and CO2 and a hydrogen stream B' comprising essentially H2, CO, CH4 and optionally N2,
f) introducing the recovered stream C"' into step d), and
g) separating hydrogen stream B' into a purified hydrogen feedstock and a separated CO, CH4 and optionally N2 stream.
